# EUROPEAN PATENT APPLICATION

(11) **EP 4 725 939 A1**
(43) Date of publication of application: **15.04.2026**
(21) Application number: 24819264.3
(22) Date of filing: 31.05.2024
(51) Int. Cl.: C07D 201/12, C07B 61/00, C07D 223/10, C08J 11/28

(54) **METHOD FOR PRODUCING CYCLIC LACTAM AND DEVICE FOR PRODUCING CYCLIC LACTAM**

(30) Priority: 08.06.2023 JP 2023094935
(71) Applicant: MITSUBISHI GAS CHEMICAL COMPANY, INC., Chiyoda-ku Tokyo 100-8324 (JP)
(72) Inventor: AOYAGI, Naoto, Tokyo 125-8601 (JP); TOKUDA, Yuka, Tokyo 125-8601 (JP); HIRAYAMA, Arinobu, Yokkaichi-shi, Mie 510-0886 (JP)
(74) Representative: Vossius & Partner Patentanwälte Rechtsanwälte mbB
(86) International application number: PCT/JP2024/020008
(87) International publication number: WO 2024/253034

(57) **Abstract**

Provided is a method for producing a cyclic lactam that can produce a cyclic lactam with good yield, as well as an apparatus for producing a cyclic lactam. A method for producing a cyclic lactam, wherein a polyamide resin comprising a repeating unit represented by formula (a) is subjected to a depolymerization reaction using a compound represented by formula (b). In formula (a), n1 is an integer of 3 to 22. The value of n1 may vary for each repeating unit. In formula (b), R¹ to R³ are each independently a hydrogen atom or an aliphatic group having 1 to 22 carbon atoms; at least one of R¹ to R³ is an aliphatic group having 5 to 22 carbon atoms; and the aliphatic group may each independently comprise at least one of the groups represented by formulae (b×1) to (b×4).

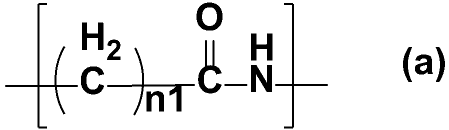

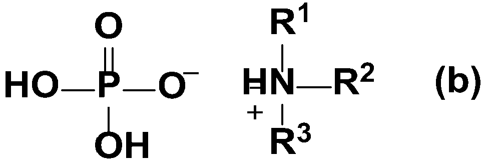

## Description

### Technical Field

The present invention relates to a method for producing a cyclic lactam, and an apparatus for producing a cyclic lactam. In particular, the present invention relates to a method for producing a cyclic lactam by a depolymerization reaction of a polyamide resin, and the like.

### Background Art

The depolymerization of polyamide resins to collect cyclic lactams has been studied in the prior art.

For example, Patent Literature 1 discloses a method for collecting ε-caprolactone and ε-caprolactam from a mixture comprising oligomers of ε-oxocapronamide and ε-caprolactam, wherein the mixture is brought into contact with water vapor at a temperature of 200 to 360°C in the presence of a phosphoric acid.

In addition, Patent Literature 2 discloses a method for purifying ε-caprolactam, wherein a thermoplastic material mainly composed of nylon 6 is depolymerized to obtain collected ε-caprolactam, and the collected ε-caprolactam is subjected to rectification.

### Citation List

### Patent Literature

Patent Literature 1: Japanese Patent Laid-Open No. S49-132094
Patent Literature 2: Japanese Patent Laid-Open No. H08-217746

### Summary of Invention

### Technical Problem

Here, Patent Literature 1 describes the use of phosphoric acids such as orthophosphoric acid, orthophosphoric acid, pyrophosphoric acid, and ammonium phosphate salts as a catalyst in the depolymerization of polyamide resins. Patent Literature 2 also describes the depolymerization of polyamide resins using phosphoric acid. However, upon investigation by the present inventor, it was found that when such phosphoric acids are used, the yield of the product, caprolactam, is low.

The present invention aims to solve such problem, and aims to provide a method for producing a cyclic lactam that can produce a cyclic lactam with good yield, as well as an apparatus for producing a cyclic lactam.

### Solution to Problem

Based on the above problem, the present inventor has conducted studies and as a result, has found that the above problem is solved by carrying out the depolymerization reaction of a polyamide resin using an ammonium phosphate salt.

Specifically, the above problem was solved by the following means.
<1> A method for producing a cyclic lactam, wherein a polyamide resin comprising a repeating unit represented by formula (a) is subjected to a depolymerization reaction using a compound represented by formula (b). 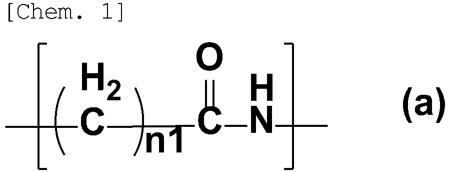 (In formula (a), n1 is an integer of 3 to 22. The value of n1 may vary for each repeating unit.) 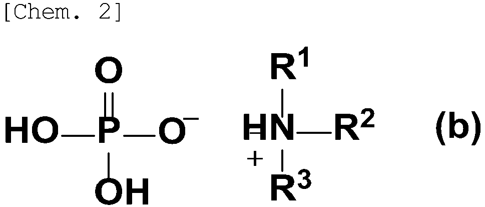 (In formula (b), R¹ to R³ are each independently a hydrogen atom or an aliphatic group having 1 to 22 carbon atoms; at least one of R¹ to R³ is an aliphatic group having 5 to 22 carbon atoms; and the aliphatic group may each independently comprise at least one of the groups represented by formulae (b×1) to (b×4).) 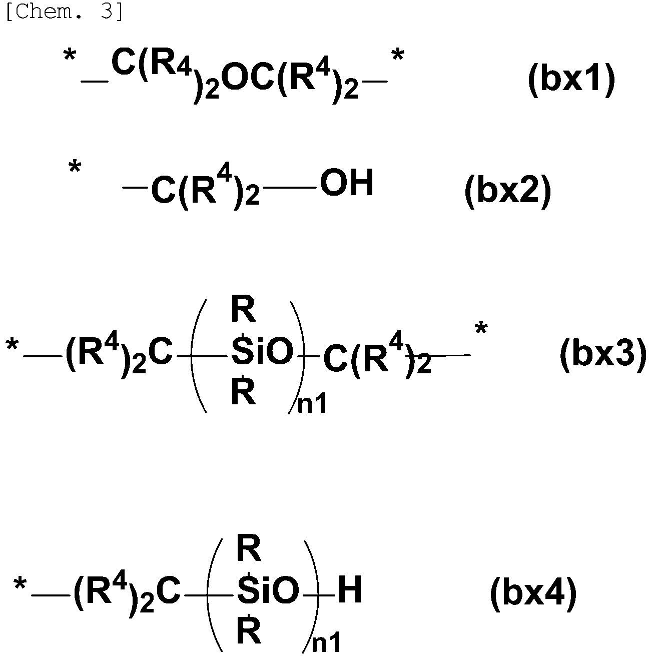 (In formulae (b×1) to (b×4), R is each independently a hydrogen atom, a methyl group, or a phenyl group; R⁴ is each independently a hydrogen atom or a methyl group; and n1 is each independently an integer of 1 to 150. * is a site of attachment to another moiety.)
<2> The method for producing a cyclic lactam according to <1>, wherein the compound composed of formula (b1) among formula (b) has a boiling point equal to or higher than the boiling point -10°C of the cyclic lactam to be produced. 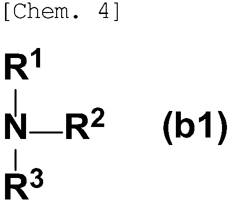 (In formula (b1), R¹ to R³ are each independently the same as R¹ to R³ in formula (b).)
<3> The method for producing a cyclic lactam according to <1> or <2>, wherein at least two of R¹ to R³ are each independently an aliphatic group having 5 to 22 carbon atoms, and the aliphatic group optionally comprises at least one of the groups represented by formulae (b×1) to (b×4).
<4> The method for producing a cyclic lactam according to any one of <1> to <3>, wherein R¹ to R³ are each independently an aliphatic group having 5 to 22 carbon atoms, and the aliphatic group optionally comprises at least one of the groups represented by formulae (b×1) to (b×4).
<5> The method for producing a cyclic lactam according to any one of <1> to <3>, wherein the aliphatic group having 5 to 22 carbon atoms represented by R¹ to R³ is an alkyl group having 5 to 22 carbon atoms.
<6> The method for producing a cyclic lactam according to any one of <1> to <5>, wherein 1 part by mass or more of the compound represented by formula (b) is used based on 100 parts by mass of the polyamide resin.
<7> The method for producing a cyclic lactam according to any one of <1> to <6>, wherein 40 to 10,000 parts by mass of the compound represented by formula (b) is used based on 100 parts by mass of the polyamide resin.
<8> The method for producing a cyclic lactam according to any one of <1> to <7>, wherein the depolymerization reaction is carried out at a pressure of 202.6 kPa or less.
<9> The method for producing a cyclic lactam according to any one of <1> to <8>, wherein the depolymerization reaction is carried out while performing reactive distillation.
<10> The method for producing a cyclic lactam according to any one of <1> to <9>, wherein distillation is performed after carrying out the depolymerization reaction.
<11> The method for producing a cyclic lactam according to any one of <1> to <10>, wherein the depolymerization reaction is carried out at a temperature of 170°C to 300°C.
<12> The method for producing a cyclic lactam according to any one of <1> to <11>, wherein superheated water vapor is not used when carrying out the depolymerization reaction.
<13> The method for producing a cyclic lactam according to any one of <1> to <12>, wherein, when carrying out the depolymerization reaction, either no solvent is used, or the amount of the solvent is 10 mass% or less based on the amount of the compound represented by formula (b).
<14> The method for producing a cyclic lactam according to any one of <1> to <13>, wherein the depolymerization reaction is carried out in a presence of a high-boiling-point solvent having a boiling point equal to or higher than the boiling point -10°C of the cyclic lactam as the product.
<15> The method for producing a cyclic lactam according to <14>, wherein the high-boiling-point solvent is not an alcohol.
<16> The method for producing a cyclic lactam according to any one of <1> to <15>, wherein the depolymerization reaction is carried out while performing reactive distillation;
   the depolymerization reaction is carried out at a temperature of 170°C to 300°C;
   superheated water vapor is not used when carrying out the depolymerization reaction; and
   when carrying out the depolymerization reaction, either no solvent is used, or the amount of the solvent is 10 mass% or less based on the amount of the compound represented by formula (b).
<17> The method for producing a cyclic lactam according to any one of <1> to <15>, wherein the depolymerization reaction is carried out while performing reactive distillation;
   the depolymerization reaction is carried out at a temperature of 170°C to 300°C;
   superheated water vapor is not used when carrying out the depolymerization reaction;
   the depolymerization reaction is carried out in a presence of a high-boiling-point solvent having a boiling point equal to or higher than the boiling point -10°C of the cyclic lactam as the product; and
   the high-boiling-point solvent is not an alcohol.
<18> The method for producing a cyclic lactam according to any one of <1> to <15>, wherein distillation is performed after carrying out the depolymerization reaction;
   the depolymerization reaction is carried out at a temperature of 170°C to 300°C;
   superheated water vapor is not used when carrying out the depolymerization reaction; and
   when carrying out the depolymerization reaction, either no solvent is used, or the amount of the solvent is 10 mass% or less based on the amount of the compound represented by formula (b).
<19> The method for producing a cyclic lactam according to any one of <1> to <15>, wherein distillation is performed after carrying out the depolymerization reaction;
   the depolymerization reaction is carried out at a temperature of 170°C to 300°C;
   superheated water vapor is not used when carrying out the depolymerization reaction;
   the depolymerization reaction is carried out in a presence of a high-boiling-point solvent having a boiling point equal to or higher than the boiling point -10°C of the cyclic lactam as the product; and
   the high-boiling-point solvent is not an alcohol.
<20> The method for producing a cyclic lactam according to any one of <1> to <19>, wherein the polyamide resin is at least one selected from the group consisting of nylon 4, nylon 5, nylon 6, nylon 11, and nylon 12.
<21> The method for producing a cyclic lactam according to any one of <1> to <20>, wherein the polyamide resin is continuously supplied to a reaction system to produce a cyclic lactam.
<22> The method for producing a cyclic lactam according to any one of <1> to <21>, wherein the compound represented by formula (b) is supplied directly to a reaction system.
<23> The method for producing a cyclic lactam according to <22>, wherein the water content of the compound represented by formula (b) supplied to the reaction system is 100 mass% or less based on the mass of the compound represented by formula (b).
<24> The method for producing a cyclic lactam according to <22> or <23>, wherein a multimer (B2) of the compound represented by formula (b) supplied to the reaction system is hydrolyzed to obtain a regenerated compound (B3) represented by formula (b) in the reaction system.
<25> The method for producing a cyclic lactam according to <24>, wherein the regenerated compound (B3) represented by formula (b) is used as the compound represented by formula (b) supplied to the reaction system.
<26> An apparatus for producing a cyclic lactam, having the following equipment for carrying out the method for producing a cyclic lactam according to any one of <1> to <25>:
   (1) a reactor for heating a mixture A comprising the polyamide resin comprising a repeating unit represented by formula (a) and the compound represented by formula (b) to carry out a depolymerization reaction and produce a cyclic lactam, thereby obtaining a mixture B comprising the compound represented by formula (b) and the cyclic lactam;
   (2) a container for accommodating the cyclic lactam evaporated from the mixture B in the reactor; and
   (3) a flow path connecting the reactor and the container, for delivering the evaporated cyclic lactam from the reactor to the container.
<27> The apparatus for producing a cyclic lactam according to <26>, further comprising a pressure regulating device for maintaining the pressure in the reactor, the container, and the flow path at 202.6 kPa or less.
<28> The apparatus for producing a cyclic lactam according to <27>, wherein the pressure regulating device is connected to at least one selected from the group consisting of the reactor, the container, and the flow path.
<29> The apparatus for producing a cyclic lactam according to any one of <26> to <28>, having a heating part for maintaining the temperature in the flow path at or above the melting point of the cyclic lactam.
<30> The apparatus for producing a cyclic lactam according to any one of <26> to <29>, having an apparatus for removing moisture from the reactor.

### Advantageous Effects of Invention

The present invention has made it possible to provide a method for producing a cyclic lactam that can produce a cyclic lactam with good yield, as well as an apparatus for producing a cyclic lactam.

### Brief Description of Drawings

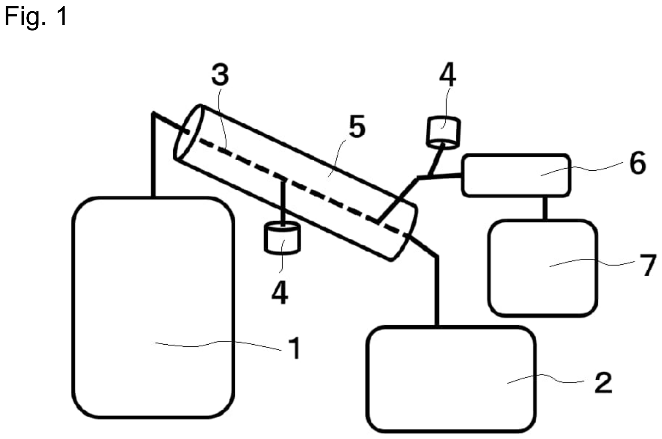

[Figure 1] Figure 1 is a schematic diagram of an example of the apparatus for producing a cyclic lactam of the present embodiment.

### Description of Embodiments

Hereinafter, an embodiment of the present invention (hereinafter, simply referred to as "the present embodiment") will be described in detail. It should be noted that the following present embodiment is an example for explaining the present invention, and the present invention is not limited only to the present embodiment.

In the present specification, "to" is used in the sense of including the numerical values listed before and after it as lower and upper limits.

Unless otherwise specified, the boiling point in the present invention means the boiling point at a pressure of 101.33 kPa.

In the present specification, the term "step" encompasses not only independent steps, but also cases where the step cannot be clearly distinguished from other steps, provided that the intended function of the step is achieved.

If the measurement methods and the like described in the standards shown herein differ from year to year, the standards as of January 1, 2023 shall be used unless otherwise specified.

Figure 1 is a schematic diagram, and the scale and the like may not correspond to the actual scale.

The method for producing a cyclic lactam of the present embodiment is characterized in that a polyamide resin comprising a repeating unit represented by formula (a) (hereinafter sometimes referred to in the present specification as "polyamide resin (a)") is subjected to a depolymerization reaction using a compound represented by formula (b). 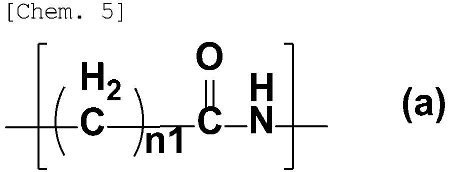 (In formula (a), n1 is an integer of 3 to 22. The value of n1 may vary for each repeating unit.) 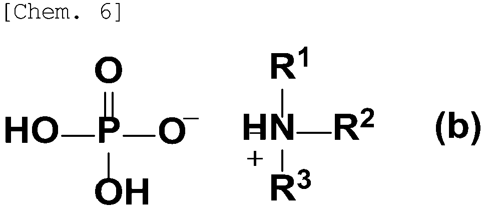 (In formula (b), R¹ to R³ are each independently a hydrogen atom or an aliphatic group having 1 to 22 carbon atoms; at least one of R¹ to R³ is an aliphatic group having 5 to 22 carbon atoms; and the aliphatic group may each independently comprise at least one of the groups represented by formulae (b×1) to (b×4).) 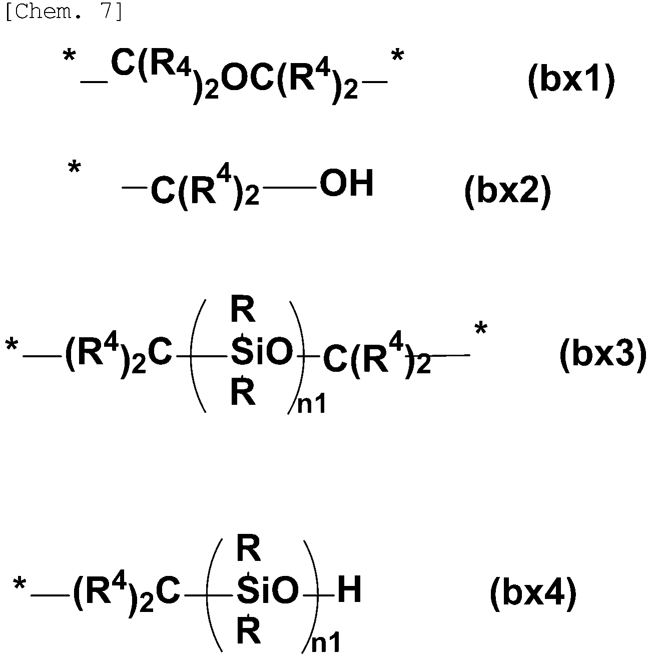 (In formulae (b×1) to (b×4), R is each independently a hydrogen atom, a methyl group, or a phenyl group; R⁴ is each independently a hydrogen atom or a methyl group; and n1 is each independently an integer of 1 to 150. * is a site of attachment to another moiety.)

Using such a configuration allows to produce a cyclic lactam with good yield.

In the method for producing a cyclic lactam of the present embodiment, the compound represented by formula (b) is usually in a liquid state at the depolymerization temperature of the polyamide resin (a) (for example, 170 to 300°C), and the depolymerization reaction proceeds with the polyamide resin (a) in a dissolved state. In other words, rather than hydrolysis mediated by water, it is presumed that a backbiting reaction proceeds, in which the terminal amino group or an amide bond in the main chain of the polyamide resin (a) attacks an amide bond adjacent by one unit to itself. In addition, since the compound represented by formula (b) has mild acidity, its bonding strength with amino groups is weaker than that of common acids, making it easier to form a cyclic amide structure during the depolymerization reaction. In other words, it is presumed that a cyclic lactam can be produced with good yield because the compound represented by formula (b) serves both as a solvent and an acid catalyst.

In particular, since the compound represented by formula (b) can also function as a solvent, the reaction proceeds efficiently without using solvents having lower boiling points than the cyclic lactam, such as water or alcohols, and the cyclic lactam produced can be purified by a simple step such as single distillation.

The following describes in detail the present invention.

### <Polyamide resin comprising repeating unit represented by formula (a) (Polyamide resin (a))>

In the present embodiment, the polyamide resin (a) used in the depolymerization reaction comprises a repeating unit represented by formula (a). 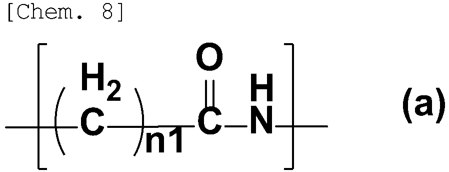 (In formula (a), n1 is an integer of 3 to 22. The value of n1 may vary for each repeating unit.)

In formula (a), n1 is preferably 4 or more, more preferably 5 or more; and preferably 20 or less, more preferably 18 or less, even more preferably 16 or less, still more preferably 14 or less, even more preferably 12 or less, yet more preferably 10 or less, further still more preferably 8 or less, and particularly more preferably 6 or less.

The proportion of the repeating unit represented by formula (a) in the polyamide resin (a) is preferably 80 mass% or more, more preferably 90 mass% or more, even more preferably 95 mass% or more, and still more preferably 99 mass% or more of the total repeating units of the polyamide resin (a).

In addition, the polyamide resin (a) may have -NH₂ and/or - COOH ends, or may be capped with an end capping agent.

The polyamide resin (a) is preferably at least one selected from the group consisting of nylon 4, nylon 5, nylon 6, nylon 11, and nylon 12, and is more preferably nylon 6.

The polyamide resin (a) may be synthesized from a cyclic lactam or an aminocarboxylic acid, but it is preferably synthesized from a cyclic lactam. In addition, nylon 6 in the present embodiment also encompasses those in which a portion thereof (for example, 5 mass% or less, furthermore 3 mass% or less, and particularly 1 mass% or less) is modified with additional monomers. The same applies to the other nylons.

In the method for producing a cyclic lactam of the present embodiment, the polyamide resin (a) may be used alone or in combination of two or more thereof.

The viscosity average molecular weight of the polyamide resin (a) is preferably 3,000 or more, more preferably 5,000 or more, even more preferably 10,000 or more, and may be 50,000 or more; and is preferably 1,000,000 or less, more preferably 500,000 or less, and even more preferably 100,000 or less.

The viscosity average molecular weight is measured in accordance with JIS K7367.

As the polyamide resin (a) used in the present embodiment, scrap or defective products of polyamide resin products, post-consumer materials, recycled materials, and the like can be utilized. In addition, the form of the polyamide resin products is not particularly limited, and, for example, fibers (drawn yarns, undrawn yarns), chips, films, and formed articles can be used. These can be appropriately processed into shapes and sizes that are easy to use in the depolymerization reaction.

In the method for producing a cyclic lactam of the present embodiment, the starting materials charged into the reaction system may include, in addition to the polyamide resin comprising the repeating unit represented by formula (a), polyamide resins which do not comprise the repeating unit represented by formula (a) (other polyamide resins). Examples of other polyamide resins include nylon 66, nylon 666, and semi-aromatic polyamide resins. However, in the method for producing a cyclic lactam of the present embodiment, the amount of polyamide resins other than the polyamide resin (a) supplied to the reaction system is preferably 5 mass% or less, more preferably 3 mass% or less, and even more preferably 1 mass% or less of the polyamide resin (a).

In addition, in the method for producing a cyclic lactam of the present embodiment, the starting materials charged into the reaction system may include, in addition to the polyamide resin (a), a thermoplastic resin other than a polyamide resin. Examples of other thermoplastic resins include polyolefin resins such as polypropylene resins and polyethylene resins, and polyester resins such as polyethylene terephthalate resins. In the method for producing a cyclic lactam of the present embodiment, the amount of other thermoplastic resin supplied to the reaction system may be, for example, 5 mass% or less, furthermore 3 mass% or less, and particularly 1 mass% or less of the polyamide resin (a).

In the method for producing a cyclic lactam of the present embodiment, the starting materials charged into the reaction system may include, in addition to the polyamide resin (a), various resin additives and fillers.

In the present embodiment, when the starting materials charged into the reaction system include various resin additives and fillers, these may be separated and collected.

For example, when the filler is water-soluble or consists of particles dispersible in water, a hydrophobic compound represented by formula (b), such as tri-n-octyl ammonium dihydrogen phosphate, can be selected as the catalyst. After completion of the reactive distillation, the filler can be extracted by adding water to the reactor 1 of Figure 1 described below, and collected from a valve attached to an appropriate position on the reactor 1. When the resin additive or filler is soluble in an organic solvent, a compound represented by formula (b) that is water-soluble or insoluble in that organic solvent can be selected as the catalyst, and after completion of the reaction, the resin additive or filler can be collected using the organic solvent.

### <Compound represented by formula (b)>

In the method for producing a cyclic lactam of the present embodiment, a polyamide resin comprising a repeating unit represented by formula (a) is subjected to a depolymerization reaction using a compound represented by formula (b).

The compound represented by formula (b) acts as a catalyst in the ring-closing reaction of the amide bonds in the polyamide resin (a), and also dissolves or disperses the polyamide resin (a), thereby contributing to a reduction in the viscosity of the reaction liquid. The reaction liquid becomes thus easier to stir, allowing to efficiently obtain the cyclic lactam. The compound represented by formula (b) is preferably directly supplied to the reaction system (for example, a reaction vessel). 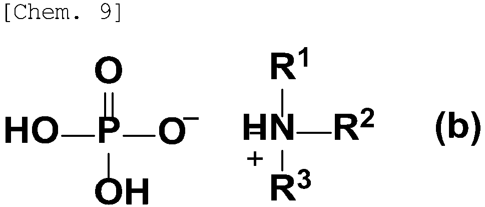 (In formula (b), R¹ to R³ are each independently a hydrogen atom or an aliphatic group having 1 to 22 carbon atoms; at least one of R¹ to R³ is an aliphatic group having 5 to 22 carbon atoms; and the aliphatic group may each independently comprise at least one of the groups represented by formulae (b×1) to (b×4).) 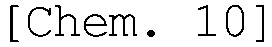 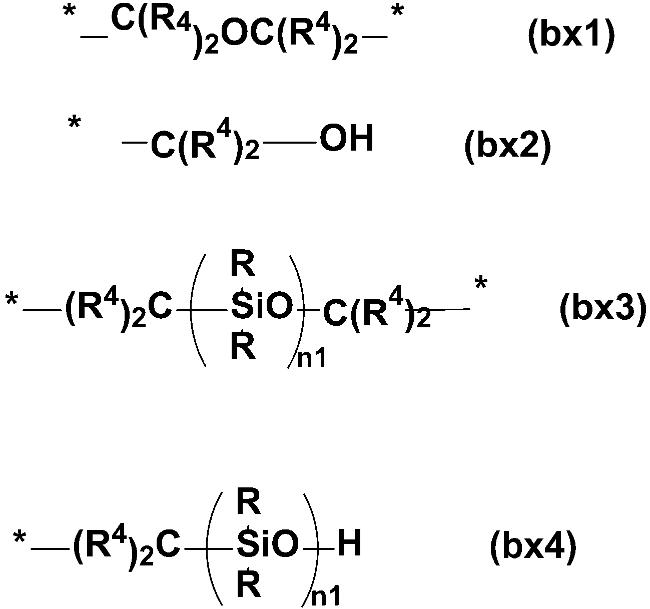 (In formulae (b×1) to (b×4), R is each independently a hydrogen atom, a methyl group, or a phenyl group; R⁴ is each independently a hydrogen atom or a methyl group; and n1 is each independently an integer of 1 to 150. * is a site of attachment to another moiety.)

In formula (b), R¹ to R³ are each independently a hydrogen atom or an aliphatic group having 1 to 22 carbon atoms, and at least one of R¹ to R³ is an aliphatic group having 5 to 22 carbon atoms. The aliphatic groups (aliphatic group having 1 to 22 carbon atoms and aliphatic group having 5 to 22 carbon atoms) may each independently comprise at least one of the groups represented by formulae (b×1) to (b×4). In one example of the present embodiment, the aliphatic group does not comprise groups represented by formulae (b×1) to (b×4).

In formula (b), R¹ to R³ are each independently a hydrogen atom or an aliphatic group having 1 to 22 carbon atoms, and are preferably each independently an aliphatic group having 1 to 22 carbon atoms.

The number of carbon atoms of the aliphatic groups represented by R¹ to R³ is preferably 3 or more, more preferably 5 or more, even more preferably 6 or more, still more preferably 7 or more, and yet more preferably 8 or more; it is preferably 20 or less, more preferably 18 or less, even more preferably 16 or less, still more preferably 14 or less, yet more preferably 12 or less, and further still more preferably 10 or less; and may be 9 or less or 8 or less.

The aliphatic groups represented by R¹ to R³ may be any of linear, branched, or cyclic aliphatic groups, or combinations thereof; and are preferably linear and/or branched aliphatic groups, and more preferably linear aliphatic groups.

The aliphatic groups represented by R¹ to R³ are preferably alkyl groups, and more preferably alkyl groups having 5 to 22 carbon atoms. Specific examples of alkyl groups having 5 to 22 carbon atoms include a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, a 1-methylhexyl group, a 2-ethylhexyl group, a 1-methylheptyl group, a 2-butyloctyl group, a 2-hexyldecyl group, a 2-octyldodecyl group, and a 2-decyltetradecyl group.

In formula (b), at least one of R¹ to R³ is an aliphatic group having 5 to 22 carbon atoms (preferably an alkyl group having 5 to 22 carbon atoms), and the aliphatic group optionally comprises at least one of the groups represented by formulae (b×1) to (b×4); it is preferable that at least two of R¹ to R³ be each independently an aliphatic group having 5 to 22 carbon atoms, and that the aliphatic group optionally comprises at least one of the groups represented by formulae (b×1) to (b×4); and it is more preferable that all of R¹ to R³ be each independently an aliphatic group having 5 to 22 carbon atoms, and that the aliphatic group optionally comprises at least one of the groups represented by formulae (b×1) to (b×4).

As described above, the aliphatic groups represented by R¹ to R³ may each independently comprise at least one of the groups represented by formulae (b×1) to (b×4). However, it is preferable that they comprise one of the groups represented by formulae (b×1) to (b×4), or include none of the groups represented by formulae (b×1) to (b×4), and it is more preferable that they comprise none of the groups represented by formulae (b×1) to (b×4). 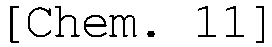 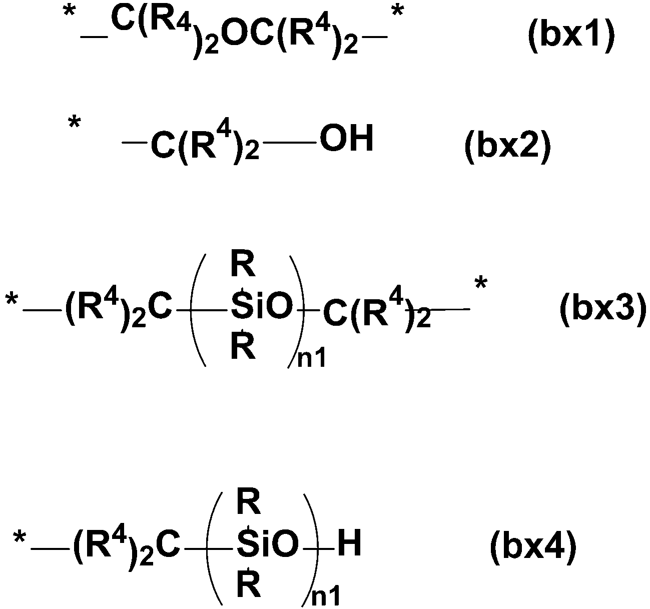 (In formulae (b×1) to (b×4), R is each independently a hydrogen atom, a methyl group, or a phenyl group; R⁴ is each independently a hydrogen atom or a methyl group; and n1 is each independently an integer of 1 to 150. * is a site of attachment to another moiety.)

In formulae (b×1) to (b×4), R is each independently a hydrogen atom, a methyl group, or a phenyl group, and is preferably a methyl group.

In formulae (b×1) to (b×4), R⁴ is each independently a hydrogen atom or a methyl group, and is preferably a hydrogen atom.

In formulae (b×1) to (b×4), n1 is each independently an integer of 1 to 150, preferably an integer of 3 or more, and preferably an integer of 100 or less, more preferably an integer of 50 or less. When n1 is 2 or more, each of the repeating units may be the same or different.

In formulae (b×1) to (b×4), * is a site of attachment to another moiety. The formulae (b×1) to (b×4) are preferably either comprised in the longest chain (main chain) of the aliphatic groups of R¹ to R³, or are bonded to the end.

In the present embodiment, in the compound represented by formula (b), R¹ to R³ in formula (b) are preferably each independently an alkyl group having 5 to 22 carbon atoms. The alkyl group having 5 to 22 carbon atoms is as described above.

In the present embodiment, the boiling point of the compound composed of formula (b1) among formula (b) preferably has a boiling point equal to or higher than the boiling point -10°C of the cyclic lactam to be produced, more preferably equal to or higher than the boiling point +10°C of the cyclic lactam, even more preferably equal to or higher than the boiling point +50°C of the cyclic lactam, and still more preferably equal to or higher than the boiling point +90°C of the cyclic lactam. By setting the boiling point at the above lower limit or more, even if a portion of the compound composed of formula (b1) is released from the catalyst during the depolymerization reaction, the proportion of the compound composed of formula (b1) that is mixed into the cyclic lactam to be distilled and purified can be reduced, which tends to increase the purity of the cyclic lactam to be distilled and purified. In addition, the boiling point of the compound composed of formula (b1) has a boiling point equal to or lower than +500°C of the cyclic lactam to be produced, and more preferably equal to or lower than the boiling point +300°C of the cyclic lactam. By setting the boiling point at the above upper limit or less, the viscosity remains low enough not to interfere with the depolymerization reaction, which tends to efficiently produce the cyclic lactam.

In the present embodiment, when two or more compounds represented by formula (b) are used, or when two or more compounds composed of formula (b1) are present, the boiling point of the compounds composed of formula (b1) is defined as the weighted average of their respective boiling points. 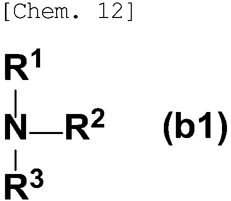 (In formula (b1), R¹ to R³ are each independently the same as R¹ to R³ in formula (b).)

The molecular weight of the compound represented by formula (b) is preferably 280 or more and more preferably 450 or more. By setting the molecular weight at the above lower limit or more, the depolymerization reaction is more effectively promoted due to high catalytic activity, which tends to allow the cyclic lactam to be produced with better yield. In addition, the molecular weight of the compound represented by formula (b) is preferably 100,000 or less, more preferably 50,000 or less, even more preferably 30,000 or less, still more preferably 10,000 or less, and yet more preferably 5,000 or less. By setting the molecular weight at the above upper limit or less, the viscosity of the reaction liquid becomes more appropriate, which tends to further improve the dissolution of the polyamide resin (a) and the efficiency of the depolymerization reaction.

Specific examples of the compound represented by formula (b) include tri-n-octyl ammonium dihydrogen phosphate and N,N-didodecylmethylammonium dihydrogen phosphate.

In the method for producing a cyclic lactam of the present embodiment, the amount of the compound represented by formula (b) used (amount added to the reaction system) is preferably 1 part by mass or more, preferably 10 parts by mass or more, more preferably 40 parts by mass or more, more preferably 50 parts by mass or more, even more preferably 70 parts by mass or more, still more preferably 90 parts by mass or more, yet more preferably 100 parts by mass or more, and further still more preferably 110 parts by mass or more, based on 100 parts by mass of the polyamide resin (a). By setting the amount at the above lower limit or more, the depolymerization reaction is more effectively promoted due to high catalytic activity, which tends to allow the cyclic lactam to be produced with better yield. The amount of the compound represented by formula (b) used (amount added to the reaction system) is preferably 10,000 parts by mass or less, more preferably 1,000 parts by mass or less, even more preferably 500 parts by mass or less, still more preferably 300 parts by mass or less, and yet more preferably 250 parts by mass or less, based on 100 parts by mass of the polyamide resin (a). By setting the amount at the above upper limit or less, it is possible to reduce starting material costs and the energy required during the distillation and purification of the cyclic lactam.

In the method for producing a cyclic lactam of the present embodiment, the amount used (amount added to the reaction system) of the phosphate moiety (H₂PO₄⁻) of the compound represented by formula (b) is preferably 1 part by mass or more, preferably 5 parts by mass or more, more preferably 10 parts by mass or more, more preferably 15 parts by mass or more, even more preferably 20 parts by mass or more, still more preferably 25 parts by mass or more, and yet more preferably 30 parts by mass or more, based on 100 parts by mass of the polyamide resin (a). By setting the amount at the above lower limit or more, the depolymerization reaction is more effectively promoted due to high catalytic activity, which tends to allow the cyclic lactam to be produced with better yield. The amount used (amount added to the reaction system) of the phosphate moiety (H₂PO₄⁻) of the compound represented by formula (b) is preferably 150 parts by mass or less, more preferably 120 parts by mass or less, even more preferably less than 100 parts by mass, still more preferably 80 parts by mass or less, and yet more preferably 50 parts by mass or less, based on 100 parts by mass of the polyamide resin (a). By setting the amount at the above upper limit or less, it is possible to reduce starting material costs and the energy required during the distillation and purification of the cyclic lactam, as well as to suppress corrosion of the apparatus.

When the polyamide resin (a) is continuously supplied to the reaction system to produce the cyclic lactam, the amount of the compound represented by formula (b), or the amount of the phosphate moiety (H₂PO₄⁻), present in the reaction system preferably falls within the above range.

In the present embodiment, the water content of the compound represented by formula (b) (the compound represented by formula (b) itself, excluding water) supplied to the reaction system is preferably 100 mass% or less, more preferably 50 mass% or less, even more preferably 10 mass% or less, still more preferably 1 mass% or less, yet more preferably 0.1 mass% or less, and further still more preferably 0.001 mass% or less, based on the mass of the compound represented by formula (b). By setting the water content at the above upper limit or less, it is possible to achieve selective production of the cyclic lactam and simple distillation and purification thereof. The lower limit of the water content of the compound represented by formula (b) supplied to the reaction system may be 0 mass%, but, for example, even 0.0001 mass% or more is sufficient to satisfy the required performance.

### <Solvent>

In the method for producing a cyclic lactam of the present embodiment, the compound represented by formula (b) serves to dissolve or disperse the polyamide resin (a), and therefore a solvent (reaction solvent) may or may not be used.

A first mode of the solvent in the method for producing a cyclic lactam of the present embodiment is a mode in which, when carrying out the depolymerization reaction of the polyamide resin (a), either no solvent is used, or the amount of the solvent is 10 mass% or less based on the amount of the compound represented by formula (b). In the first mode of the solvent, even in a configuration that substantially does not use a solvent, the compound represented by formula (b) also serves as a solvent. In the first mode of the solvent, the amount of the solvent is preferably 5 mass% or less, more preferably 3 mass% or less, even more preferably 1 mass% or less, and still more preferably 0.1 mass% or less, based on the amount of the compound represented by formula (b).

In the first mode of the solvent described above, purification of the cyclic lactam by reactive distillation or by distillation after the reaction can be readily performed. Energy costs also tend to be reduced.

In the first mode of the solvent described above, it is preferable that 95 mass% or more (preferably 99 mass% or more) of the substances supplied to the reaction system (for example, a reaction vessel) are composed of the compound represented by formula (b) and the polyamide resin (a). However, it goes without saying that, during the reaction, in addition to the cyclic lactam as the target product, water that is generated, unreacted polyamide resin (a), and polyamide oligomers as intermediates are also present in the reaction system.

A second mode of the solvent in the method for producing a cyclic lactam of the present embodiment is a mode in which the depolymerization reaction of the polyamide resin (a) is carried out in the presence of a high-boiling-point solvent having a boiling point equal to or higher than the boiling point -10°C of the cyclic lactam as the product. By using a high-boiling-point solvent, the viscosity of the reaction liquid is reduced, which tends to promote the depolymerization reaction, and improve the yield of the product distilled and purified (cyclic lactam).

The boiling point of the high-boiling-point solvent is preferably equal to or higher than the boiling point -5°C of the cyclic lactam as the product, more preferably equal to or higher than the boiling point -3°C, even more preferably equal to or higher than the boiling point -1°C, still more preferably the boiling point or higher, yet more preferably equal to or higher than the boiling point +1°C, and further still more preferably equal to or higher than the boiling point +3°C. The upper limit of the boiling point of the high-boiling-point solvent is preferably equal to or lower than the boiling point +200°C of the cyclic lactam as the product.

In addition, when two or more high-boiling-point solvents are comprised, the boiling point of the solvent present in the largest amount is used.

The viscosity of the high-boiling-point solvent at 25°C is preferably 1 cSt or more, more preferably 10 cSt or more, and may be 20 cSt or more; and is preferably 500 cSt or less, more preferably 300 cSt or less, and even more preferably 100 cSt or less. The viscosity of the high-boiling-point solvent is measured in accordance with JIS Z8803.

Examples of the high-boiling-point solvent include polysiloxanes and end-capped polyethylene glycols, with polyalkylsiloxanes (the alkyl groups having 1 to 3 carbon atoms, with an average of one or two alkyl groups bonded to a single Si atom) being preferred, and polydimethylsiloxane being more preferred.

In addition, the high-boiling-point solvent is preferably not an alcohol. By not using an alcohol, a decrease in catalytic activity of the compound represented by formula (b) due to alcoholysis can be effectively prevented.

In the second mode of the solvent in the method for producing a cyclic lactam of the present embodiment, the amount of the high-boiling-point solvent used is preferably 10 parts by mass or more, more preferably 50 parts by mass or more, and even more preferably 100 parts by mass or more, based on 100 parts by mass of the compound represented by formula (b). By setting the amount at the above lower limit or more, the depolymerization reaction is promoted, which tends to improve the yield of the product distilled and purified (cyclic lactam). In addition, the upper limit of the amount of the high-boiling-point solvent used is preferably 1,000 parts by mass or less, more preferably 500 parts by mass or less, even more preferably 400 parts by mass or less, still more preferably 300 parts by mass or less, and even more preferably 200 parts by mass or less, based on 100 parts by mass of the compound represented by formula (b). Setting the amount to the above upper limit or less tends to prevent the reaction liquid from becoming too dilute. More specifically, the starting material costs can be reduced, and the depolymerization reactivity can be maintained at a high level.

In the method for producing a cyclic lactam of the present embodiment, the high-boiling-point solvent may be used alone or in combination of two or more thereof. When two or more are used, the total amount is preferably in the above range.

In the second mode of the solvent described above, it is preferable that 95 mass% or more (preferably 99 mass% or more) of the substances supplied to the reaction system (for example, a reaction vessel) are composed of the compound represented by formula (b), the polyamide resin (a), and the high-boiling-point solvent. However, it goes without saying that, during the reaction, in addition to the cyclic lactam as the target product, water that is generated, unreacted polyamide resin (a), and polyamide oligomers as intermediate products are also present in the reaction system.

### <Depolymerization Reaction>

Next, the details of the depolymerization reaction will be described.

In the present embodiment, the pressure at which the depolymerization reaction is carried out is preferably 202.6 kPa or less, more preferably 150.0 kPa or less, even more preferably less than 101.3 kPa, still more preferably 13.3 kPa or less, and yet more preferably 4.0 kPa or less. Setting the pressure at the above upper limit or less tends to allow the cyclic lactam to be obtained with a higher yield by reactive distillation. The lower limit of the pressure at which the depolymerization reaction is carried out is preferably 0.01 kPa or more, for example.

The pressure at which the depolymerization reaction is carried out means the pressure at a steady state after being adjusted by reducing or increasing the pressure in the reaction system. In the present embodiment, for example, the reaction may be carried out under two or more pressure stages, such as reacting for a certain period of time under normal pressure and then reacting for a certain period of time under reduced pressure. In this case, the pressure at each stage preferably falls within the above range.

The temperature at which the depolymerization reaction is carried out is preferably 170°C or more, more preferably 190°C or more, even more preferably 210°C or more, and still more preferably 220°C or more. By setting the temperature at the above lower limit or more, the depolymerization reaction is promoted, which allows for more efficient distillation of the cyclic lactam. In addition, the temperature at which the depolymerization reaction is carried out is preferably 300°C or less, more preferably 280°C or less, and even more preferably 250°C or less. Setting the temperature at the above upper limit or less allows to reduce the energy required for producing the cyclic lactam, which tends to allow to reduce the formation of by-products.

The temperature at which the depolymerization reaction is carried out means the temperature at a steady state after increasing the temperature in the reaction system. In the present embodiment, the reaction may be carried out under two or more temperature stages, such as reacting for a certain period of time at a specific temperature and then further reacting for a certain period of time after increasing or reducing the temperature. In this case, the reaction temperature at each stage preferably falls within the above range.

The depolymerization reaction may be carried out without stirring, but it is preferably carried out with stirring. Stirring allows to more effectively facilitate the reaction. In addition, in the present embodiment, stirring can be performed by using the compound represented by formula (b).

The stirring rate can be appropriately adjusted according to the reaction scale (apparatus scale) and the apparatus structure, but it is preferably 10 rpm or more, more preferably 30 rpm or more, even more preferably 100 rpm or more, still more preferably 150 rpm or more, yet more preferably 200 rpm or more, and may be 300 rpm or more or 500 rpm or more. By setting the stirring rate at the above lower limit or more, the polyamide resin (a) and the compound represented by formula (b) tend to be mixed more uniformly, and the depolymerization reaction tends to proceed efficiently. The stirring rate is preferably 1,500 rpm or less, more preferably 1,200 rpm or less, even more preferably 1,000 rpm or less, still more preferably 800 rpm or less, yet more preferably 600 rpm or less, and may be 550 rpm or less. By setting the stirring rate at the above upper limit or less, splashing of the reaction liquid onto the sidewalls or top of the reaction apparatus can be suppressed, thereby effectively suppressing a decrease in yield. In addition, blockage of the distillation flow path can be effectively prevented.

The stirring rate means the temperature at a steady state after adjusting the stirring rate in the reaction system. Stirring may be performed at two or more stirring rate stages, such as reacting for a certain period of time at a specific stirring rate and then further stirring at another stirring rate. In this case, the stirring rate at each stage preferably falls within the above range.

As for the equipment used for stirring, equipment commonly used in the production of chemical products can be used, and for example, the shape of the stirring blades can be appropriately selected, such as paddle type, anchor type, or helical ribbon type.

In the present embodiment, superheated vapor may not be used when carrying out the depolymerization reaction. By not using superheated vapor, it is possible to effectively reduce the energy and waste during purification.

Examples of superheated vapor include superheated vapor of water, amines, and alcohols. More specific examples include superheated water vapor, methanol vapor, and ammonia vapor. In the present embodiment, it is particularly advantageous that the depolymerization reaction can be facilitated without using superheated water vapor.

The depolymerization reaction in the present embodiment is usually not a hydrolysis reaction mediated by water. Accordingly, in the present embodiment, it is preferable to carry out the depolymerization reaction while removing moisture.

In addition, the depolymerization reaction in the present embodiment preferably proceeds via a back-biting reaction, in which the terminal amino group or an amide bond in the main chain of the polyamide resin (a) attacks an amide bond adjacent by one unit to itself.

### <Distillation>

In the method for producing a cyclic lactam of the present embodiment, distillation is preferably performed. By performing distillation, the cyclic lactam as the target product can be isolated and purified. While the reaction system may contain water due to the starting material polyamide resin (a) absorbing moisture or due to partial condensation of the compound represented by formula (b) during the reaction, the moisture can be removed by distillation.

Distillation may be performed while carrying out the depolymerization reaction as reactive distillation, or it may be performed after carrying out the depolymerization reaction. Of course, reactive distillation may be performed while carrying out the depolymerization reaction, and distillation may also be performed after carrying out the depolymerization reaction.

The pressure at which the distillation is performed after carrying out the depolymerization reaction is preferably less than 101.3 kPa, more preferably 13.3 kPa or less, even more preferably less than 4.0 kPa, still more preferably 2.7 kPa or less, and yet more preferably 1.4 kPa or less. Setting the pressure at the above upper limit or less tends to allow the cyclic lactam to be isolated with a better yield. The lower limit of the pressure at which the distillation is performed is preferably 0.01 kPa or more, for example.

### <Supply to Reaction System>

In the method for producing a cyclic lactam of the present embodiment, the starting material polyamide resin (a) and the compound represented by formula (b) are usually supplied to a reaction system (for example, a reaction vessel) to produce the cyclic lactam.

The polyamide resin (a) may be supplied to the reaction system all at once, or it may be continuously supplied to the reaction system. When the polyamide resin (a) is continuously supplied to the reaction system, the amount of the compound represented by formula (b) present in the reaction system is preferably 1 part by mass or more based on 100 parts by mass of the polyamide resin (a).

In the method for producing a cyclic lactam of the present embodiment, the compound represented by formula (b) is supplied to the reaction system (for example, a reaction vessel), in which case it may be supplied to the reaction system all at once, or it may be continuously supplied to the reaction system. From an industrial production standpoint, continuous supply is preferred. Reuse of the compound represented by formula (b) is also preferred.

The polyamide resin (a) and the compound represented by formula (b) may each be supplied separately to the reaction system (for example, a reaction vessel), or they may be premixed and then supplied to the reaction system. In the present embodiment, the polyamide resin (a) and the compound represented by formula (b) are preferably each directly supplied to the reaction system (for example, a reaction vessel).

The compound represented by formula (b) supplied to the reaction system may undergo oligomerization as the production of the cyclic lactam proceeds. Therefore, the method for producing a cyclic lactam of the present embodiment may include hydrolyzing the multimer (B2) of the compound represented by formula (b) supplied to the reaction system, to obtain the regenerated compound (B3) represented by formula (b).

In the present embodiment, in particular, it is also possible to hydrolyze the multimer (B2) of the compound represented by formula (b) supplied to the reaction system, to obtain the regenerated compound (B3) represented by formula (b) in the reaction system.

Furthermore, the regenerated compound (B3) represented by formula (b) can also be used as the compound represented by formula (b) supplied to the reaction system.

Here, examples of the multimer (B2) obtained by the oligomerization of the compound represented by formula (b) supplied to the reaction system include a multimer in which the phosphate portion has polymerized.

### <Embodiments>

Preferred embodiments of the method for producing a cyclic lactam are described below. It goes without saying that the present invention is not limited to these embodiments.

A first embodiment of the method for producing a cyclic lactam is a method for producing a cyclic lactam, in which the depolymerization reaction is carried out while performing reactive distillation; the depolymerization reaction is carried out at a temperature of 170°C to 300°C; superheated water vapor is not used when carrying out the depolymerization reaction; and when carrying out the depolymerization reaction, either no solvent is used or the amount of the solvent is 10 mass% or less based on the amount of the compound represented by formula (b).

By employing the first embodiment, the production time of the cyclic lactam can be shortened, which allows to efficiently obtain the cyclic lactam.

A second embodiment of the method for producing a cyclic lactam is a method for producing a cyclic lactam, in which the depolymerization reaction is carried out while performing reactive distillation; the depolymerization reaction is carried out at a temperature of 170°C to 300°C; superheated water vapor is not used when carrying out the depolymerization reaction; the depolymerization reaction is carried out in the presence of a high-boiling-point solvent having a boiling point equal to or higher than the boiling point -10°C of the cyclic lactam as the product; and the high-boiling-point solvent is not an alcohol.

By employing the second embodiment, the viscosity of the reaction liquid can be reduced, which promotes the depolymerization reaction, and the production time of the cyclic lactam can be shortened by reactive distillation.

A third embodiment of the method for producing a cyclic lactam is a method for producing a cyclic lactam, in which distillation is performed after carrying out the depolymerization reaction; the depolymerization reaction is carried out at a temperature of 170°C to 300°C; superheated water vapor is not used when carrying out the depolymerization reaction; and when carrying out the depolymerization reaction, either no solvent is used or the amount of the solvent is 10 mass% or less based on the amount of the compound represented by formula (b).

By employing the third embodiment, distillation and purification can be performed at a temperature lower than that during the depolymerization reaction.

A fourth embodiment of the method for producing a cyclic lactam is a method for producing a cyclic lactam, in which the distillation is performed after carrying out the depolymerization reaction; the depolymerization reaction is carried out at a temperature of 170°C to 300°C; superheated water vapor is not used when carrying out the depolymerization reaction; the depolymerization reaction is carried out in the presence of a high-boiling-point solvent having a boiling point equal to or higher than the boiling point -10°C of the cyclic lactam as the product; and the high-boiling-point solvent is not an alcohol.

By employing the fourth embodiment, the viscosity of the reaction liquid can be reduced, which promotes the depolymerization reaction, and distillation and purification can be performed at a temperature lower than that during the depolymerization reaction.

### <Apparatus for Producing Cyclic Lactam>

In the present embodiment, an apparatus for producing a cyclic lactam, which utilizes the method for producing a cyclic lactam of the present embodiment described above, is further disclosed. Figure 1 is a schematic diagram of an example of the apparatus for producing a cyclic lactam. In Figure 1, 1 denotes a reactor, 2 denotes a container for accommodating the cyclic lactam, 3 denotes a flow path, 4 denotes a pressure regulating device, 5 denotes a heating part, 6 denotes an apparatus for removing moisture, and 7 denotes a container for collecting water. The apparatus for producing a cyclic lactam of the present embodiment will be described below with reference to Figure 1. However, it goes without saying that the apparatus for producing a cyclic lactam of the present embodiment is not limited to Figure 1.

Specifically, the present embodiment discloses an apparatus for producing a cyclic lactam, having the following equipment for carrying out the method for producing a cyclic lactam of the present embodiment described above.
(1) a reactor 1 for heating a mixture A comprising the polyamide resin (a) and the compound represented by formula (b) to carry out a depolymerization reaction and produce a cyclic lactam, thereby obtaining a mixture B comprising the compound represented by formula (b) and the cyclic lactam;
(2) a container 2 for accommodating the cyclic lactam evaporated from the mixture B in the reactor 1; and
(3) a flow path 3 connecting the reactor 1 and the container 2, for delivering the evaporated cyclic lactam from the reactor 1 to the container 2.

The mixture B usually also comprises a polyamide resin comprising a repeating unit represented by formula (a) and/or a polyamide oligomer comprising a repeating unit represented by formula (a).

The compound represented by formula (b) in the mixture A comprising the polyamide resin (a) and the compound represented by formula (b), and in the mixture B comprising the compound represented by formula (b) and the cyclic lactam, is identical to the polyamide resin (a) and the compound represented by formula (b) supplied to the reaction system described above. The ratios, preferred ranges, and the like thereof can be determined by reference to the descriptions of <Polyamide resin comprising repeating unit represented by formula (a) (Polyamide resin (a))> and <Compound represented by formula (b)> described above. Furthermore, the mixture A and mixture B optionally comprises a solvent, and the details thereof can be referred to in the description of <Solvent>.

Details of the depolymerization reaction can be referred to in the description of <Depolymerization Reaction> described above.

Examples of the reactor 1 include those made of stainless steel with glass lining or anodic protection, or made of tantalum.

Examples of the container 2 for accommodating the cyclic lactam evaporated from the mixture B in the reactor 1 include those made of at least one selected from the group consisting of glass-lined stainless steel, stainless steel (SUS), tantalum, titanium, Hastelloy, Teflon (registered trademark) lining, nickel, zirconium, copper, and carbon steel (CS).

Examples of the flow path 3 (indicated by a dotted line in Figure 1 for convenience) connecting the reactor 1 and the container 2, for delivering the evaporated cyclic lactam from the reactor 1 to the container 2, include flow paths made of stainless steel and/or titanium.

Valves, joints, or the like may be provided between the reactor 1 and the flow path 3 and/or between the container 2 and the flow path 3. Providing a valve allows to regulate the pressure and flow rate. A valve may also be provided in the middle of the flow path 3 to regulate the pressure and flow rate.

Furthermore, the apparatus for producing a cyclic lactam of the present embodiment preferably has a pressure regulating device 4 for maintaining the pressure in the reactor 1, the container 2, and the flow path 3 at 202.6 kPa or less. Examples of the pressure regulating device 4 include vacuum pumps such as reciprocating piston pumps and rotary pumps. In the apparatus for producing a cyclic lactam of the present embodiment, the pressure in the entire region through which the starting materials or products may pass in the apparatus, including inside the reactor 1, the container 2, and the flow path 3, may be regulated by a single pressure regulating device 4 installed in any one of the reactor 1, the container 2, or the flow path 3, or the pressure may be regulated by pressure regulating devices 4 installed in each of the reactor 1, the container 2, and the flow path 3.

The pressure regulating device 4 may be connected to any part of the reactor 1, the container 2, or the flow path 3 described above, but it is preferably connected to the flow path 3. By adopting such a configuration, compounds with a lower boiling point than the cyclic lactam, such as water, which are present (in small amounts) as contaminants, can be efficiently separated. Valves, joints, or the like may be provided between the flow path 3 and the pressure regulating device 4.

In addition, the pressure regulating device 4 is also preferably connected between the flow path 3 and the apparatus 6 for removing moisture described below.

Furthermore, in the present embodiment, it is preferable to have a heating part 5 for maintaining the temperature in the flow path 3 at or above the melting point of the cyclic lactam (preferably at the melting point +1°C or higher, and preferably at the melting point +50°C or lower). By providing the heating part 5, blockage caused by deposition of the cyclic lactam can be effectively suppressed. Examples of the heating part 5 include a heat medium, such as hot water or vapor at 50°C or more (for example, less than 160°C), and a heat exchanger through which the heat medium is circulated.

In addition, in the apparatus for producing a cyclic lactam of the present embodiment, it is preferable to have an apparatus 6 for removing moisture from the reactor 1. By providing the apparatus 6 for removing moisture, hydrolysis of the cyclic lactam and the compound represented by formula (b) can be effectively suppressed. Examples of the apparatus 6 for removing moisture include a condenser and a molecular sieve column. Furthermore, the apparatus for producing a cyclic lactam of the present embodiment preferably has a container 7 for collecting the removed moisture.

The cyclic lactam obtained by the method or apparatus for producing a cyclic lactam of the present embodiment can be reused as a starting material for polyamide resins or fibers, or can be used as a starting material for medical polymers.

### Examples

Hereinafter, the present invention will be described more specifically with reference to Examples. The materials, amounts used, proportions, treatment details, treatment procedures, and the like shown in the following Examples can be appropriately modified without departing from the subject matter of the present invention. Accordingly, the scope of the present invention is not limited to the specific examples shown below.

When the measuring instruments or the like used in the Examples become unavailable due to discontinuation or the like, the measurements can be carried out using other instruments having equivalent performance.

Octyl means an octyl group.

### Starting Materials

Polyamide 6 (Nylon 6): manufactured by Sigma Aldrich, 181110, viscosity average molecular weight: 10,000.
H₃PO₄-N(Octyl)₃: tri-n-octyl ammonium dihydrogen phosphate, synthesized according to the following Synthesis Example.
H₃PO₄-NH₃: ammonium dihydrogen phosphate, manufactured by KANTO CHEMICAL CO.,INC., 01309-30.
H₃PO₄: phosphoric acid, manufactured by FUJIFILM Wako Pure Chemical Corporation, 167-02166, water content: 14.4 mass%.
H₂SO₄-N(Octyl)₃: tri-n-octyl ammonium hydrogen sulfate, synthesized according to the following Synthesis Example.
NaH₂PO₄ manufactured by KANTO CHEMICAL CO.,INC., 37403-00.

### <Synthesis Example of H₃PO₄-N(Octyl)₃>

To a three-neck flask equipped with a condenser fitted with a drying tube, a pressure-equalizing dropping funnel, and a stirring apparatus was added 10 mL of tetrahydrofuran and 1.15 g of 85 mass% phosphoric acid (manufactured by FUJIFILM Wako Pure Chemical Corporation) (10 mmol as H₃PO₄). To the pressure-equalizing dropping funnel was added 3.54 g (10 mmol) of tri-n-octylamine (N(Octyl)₃) (manufactured by Tokyo Chemical Industry Co., Ltd.) dissolved in 30 mL of tetrahydrofuran. This solution was then added dropwise over 1 hour at room temperature to the phosphoric acid-tetrahydrofuran solution under stirring at a rate of 600 rpm. The mixture was then heated and stirred in an oil bath at 80°C for 1 hour. After cooling, the solvent was removed using an evaporator. The resulting residue was vacuum-dried at 60°C for 48 hours to obtain H₃PO₄-N(Octyl)₃ as a yellow transparent syrup with a yield of 4.45 g (yield; 98.2%).

The compound was identified as H₃PO₄-N(Octyl)₃ by proton (¹H, 500 MHz), carbon (¹³C, 125 MHz), and phosphorus (³¹P, 202 MHz) nuclear magnetic resonance (NMR) spectroscopy.

### <Synthesis Example of H₂SO₄-N(Octyl)₃>

To a three-neck flask equipped with a condenser fitted with a drying tube, a pressure-equalizing dropping funnel, and a stirring apparatus was added 10 mL of tetrahydrofuran and 1.03 g of 95 mass% sulfuric acid (10 mmol as H₂SO₄). To the pressure-equalizing dropping funnel was added 3.54 g (10 mmol) of tri-n-octylamine (N(Octyl)₃) dissolved in 30 mL of tetrahydrofuran. This solution was then added dropwise over 1 hour at room temperature to the phosphoric acid-tetrahydrofuran solution under stirring at a rate of 600 rpm. The mixture was then heated and stirred in an oil bath at 80°C for 1 hour. After cooling, the solvent was removed using an evaporator. The resulting residue was vacuum-dried at 60°C for 48 hours to obtain H₂SO₄-N(Octyl)₃ as a pale yellow wax with a yield of 4.48 g (yield; 99.1%).

The compound was identified as H₂SO₄-N(Octyl)₃ by proton (¹H, 500 MHz) and carbon (¹³C, 125 MHz) nuclear magnetic resonance (NMR) spectroscopy.

### Example 1

A pear-shaped flask equipped with a condenser fitted with a drying tube and a stirring apparatus was charged with 200 parts by mass of the catalyst shown in Table 1 based on 100 parts by mass of polyamide 6. The mixture was then reacted for 6 hours at 230°C using an oil bath, under a pressure of 20 mmHg (2.67 kPa), while stirring at a rate of 600 rpm.

White crystals were obtained in a high yield of 88 mass% by reactive distillation. The compound was identified as ε-caprolactam (ε-CL) by proton nuclear magnetic resonance (¹H NMR) spectroscopy.

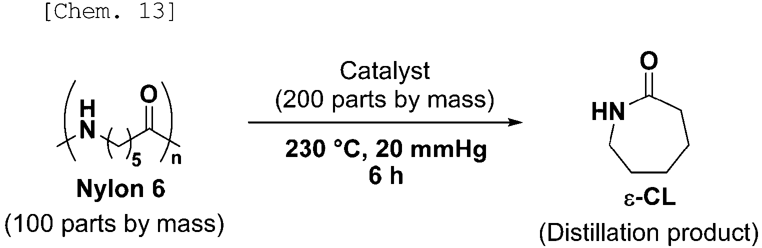

### Comparative Examples 1 to 4

As shown in Table 1, the type and amount of catalyst were modified as shown in Table 1, and the rest was carried out in the same manner as in Example 1.

The results of Example 1 and Comparative Examples 1 to 4 are summarized in Table 1 below.

**[Table 1]**

| | Catalyst | | ε-Caprolactam |
|---|---|---|---|
| | Compound Name | Parts by mass (Phosphate moiety) | Distillation yield/%^{a} |
| Example 1 | H₃PO₄-N(OCtyl)₃ | 200 (43) | 88 |
| Comparative Example 1 | H₃PO₄-NH₃ | 200 (169) | 30 |
| Comparative Example 2 | H₃PO₄ (14.4 mass% moisture) | 200 (169) | 42 |
| Comparative Example 3 | H₂SO₄-N(Octyl)₃ | 200 (0) | 4 |
| Comparative Example 4 | NaH₂PO₄ | 200 (162) | 0 |
| ^{a1}H NMR analysis | | | |

In Table 1, "parts by mass (phosphate moiety)" indicates the amount of the phosphate moiety (H₂PO₄⁻) in each catalyst added to the reaction system.

In Example 1, although the added amount of phosphate moiety directly involved in the depolymerization was markedly smaller than in Comparative Examples 1, 2, and 4, it was confirmed that the depolymerization reaction proceeded effectively. In addition, even when using an ammonium salt having an aliphatic group having 5 to 22 carbon atoms, almost no target cyclic lactam was obtained when an ammonium salt of sulfuric acid was used (Comparative Example 3).

That is, it was found that, in the present invention, carrying out the depolymerization reaction using a compound represented by formula (b) is extremely important.

In addition, in Example 1, a multimer in which the phosphate portion of the compound represented by formula (b) was polymerized was obtained.

### Reference Signs List

1 Reactor
2 Container for accommodating the cyclic lactam
3 Flow path
4 Pressure regulating device
5 Heating part
6 Apparatus for removing moisture
7 Container for collecting moisture

## Claims

1. A method for producing a cyclic lactam, wherein a polyamide resin comprising a repeating unit represented by formula (a) is subjected to a depolymerization reaction using a compound represented by formula (b). 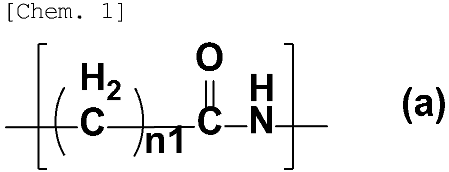 (In formula (a), n1 is an integer of 3 to 22. The value of n1 may vary for each repeating unit.) 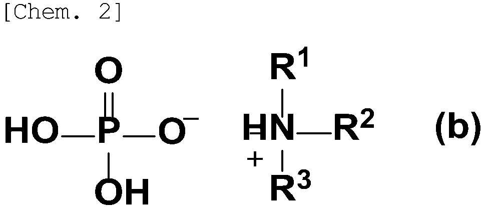 (In formula (b), R¹ to R³ are each independently a hydrogen atom or an aliphatic group having 1 to 22 carbon atoms; at least one of R¹ to R³ is an aliphatic group having 5 to 22 carbon atoms; and the aliphatic group may each independently comprise at least one of the groups represented by formulae (b×1) to (b×4).) 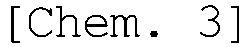 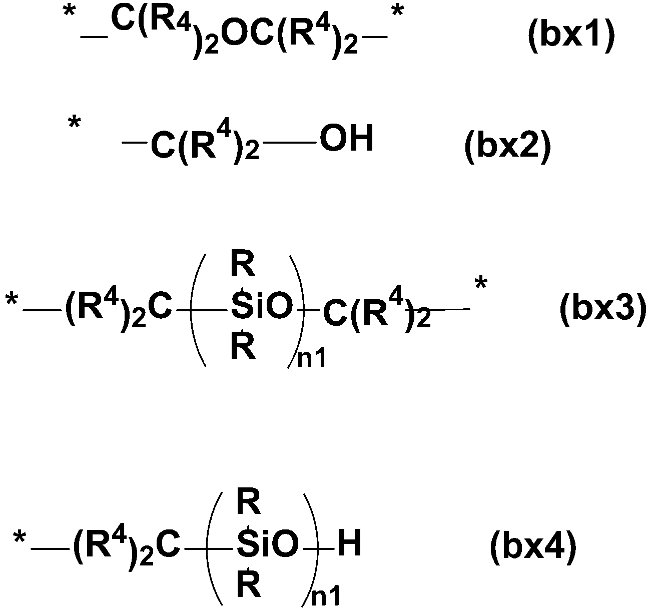 (In formulae (b×1) to (b×4), R is each independently a hydrogen atom, a methyl group, or a phenyl group; R⁴ is each independently a hydrogen atom or a methyl group; and n1 is each independently an integer of 1 to 150. * is a site of attachment to another moiety.)

2. The method for producing a cyclic lactam according to claim 1, wherein the compound composed of formula (b1) among formula (b) has a boiling point equal to or higher than the boiling point -10°C of the cyclic lactam to be produced. 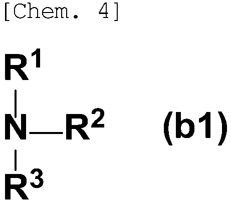 (In formula (b1), R¹ to R³ are each independently the same as R¹ to R³ in formula (b).)

3. The method for producing a cyclic lactam according to claim 1, wherein at least two of R¹ to R³ are each independently an aliphatic group having 5 to 22 carbon atoms, and the aliphatic group optionally comprises at least one of the groups represented by formulae (b×1) to (b×4).

4. The method for producing a cyclic lactam according to claim 1, wherein R¹ to R³ are each independently an aliphatic group having 5 to 22 carbon atoms, and the aliphatic group optionally comprises at least one of the groups represented by formulae (b×1) to (b×4).

5. The method for producing a cyclic lactam according to claim 1, wherein the aliphatic group having 5 to 22 carbon atoms represented by R¹ to R³ is an alkyl group having 5 to 22 carbon atoms.

6. The method for producing a cyclic lactam according to claim 1, wherein 1 part by mass or more of the compound represented by formula (b) is used based on 100 parts by mass of the polyamide resin.

7. The method for producing a cyclic lactam according to claim 1, wherein 40 to 10,000 parts by mass of the compound represented by formula (b) is used based on 100 parts by mass of the polyamide resin.

8. The method for producing a cyclic lactam according to claim 1, wherein the depolymerization reaction is carried out at a pressure of 202.6 kPa or less.

9. The method for producing a cyclic lactam according to claim 1, wherein the depolymerization reaction is carried out while performing reactive distillation.

10. The method for producing a cyclic lactam according to claim 1, wherein distillation is performed after carrying out the depolymerization reaction.

11. The method for producing a cyclic lactam according to claim 1, wherein the depolymerization reaction is carried out at a temperature of 170°C to 300°C.

12. The method for producing a cyclic lactam according to claim 1, wherein superheated water vapor is not used when carrying out the depolymerization reaction.

13. The method for producing a cyclic lactam according to claim 1, wherein, when carrying out the depolymerization reaction, either no solvent is used, or the amount of the solvent is 10 mass% or less based on the amount of the compound represented by formula (b).

14. The method for producing a cyclic lactam according to claim 1, wherein the depolymerization reaction is carried out in a presence of a high-boiling-point solvent having a boiling point equal to or higher than the boiling point -10°C of the cyclic lactam as the product.

15. The method for producing a cyclic lactam according to claim 14, wherein the high-boiling-point solvent is not an alcohol.

16. The method for producing a cyclic lactam according to claim 1, wherein the depolymerization reaction is carried out while performing reactive distillation;
the depolymerization reaction is carried out at a temperature of 170°C to 300°C;
superheated water vapor is not used when carrying out the depolymerization reaction; and
when carrying out the depolymerization reaction, either no solvent is used, or the amount of the solvent is 10 mass% or less based on the amount of the compound represented by formula (b).

17. The method for producing a cyclic lactam according to claim 1, wherein the depolymerization reaction is carried out while performing reactive distillation;
the depolymerization reaction is carried out at a temperature of 170°C to 300°C;
superheated water vapor is not used when carrying out the depolymerization reaction;
the depolymerization reaction is carried out in a presence of a high-boiling-point solvent having a boiling point equal to or higher than the boiling point -10°C of the cyclic lactam as the product; and
the high-boiling-point solvent is not an alcohol.

18. The method for producing a cyclic lactam according to claim 1, wherein distillation is performed after carrying out the depolymerization reaction;
the depolymerization reaction is carried out at a temperature of 170°C to 300°C;
superheated water vapor is not used when carrying out the depolymerization reaction; and
when carrying out the depolymerization reaction, either no solvent is used, or the amount of the solvent is 10 mass% or less based on the amount of the compound represented by formula (b).

19. The method for producing a cyclic lactam according to claim 1, wherein distillation is performed after carrying out the depolymerization reaction;
the depolymerization reaction is carried out at a temperature of 170°C to 300°C;
superheated water vapor is not used when carrying out the depolymerization reaction;
the depolymerization reaction is carried out in a presence of a high-boiling-point solvent having a boiling point equal to or higher than the boiling point -10°C of the cyclic lactam as the product; and
the high-boiling-point solvent is not an alcohol.

20. The method for producing a cyclic lactam according to claim 1, wherein the polyamide resin is at least one selected from the group consisting of nylon 4, nylon 5, nylon 6, nylon 11, and nylon 12.

21. The method for producing a cyclic lactam according to claim 1, wherein the polyamide resin is continuously supplied to a reaction system to produce a cyclic lactam.

22. The method for producing a cyclic lactam according to claim 1, wherein the compound represented by formula (b) is supplied directly to a reaction system.

23. The method for producing a cyclic lactam according to claim 22, wherein the water content of the compound represented by formula (b) supplied to the reaction system is 100 mass% or less based on the mass of the compound represented by formula (b).

24. The method for producing a cyclic lactam according to claim 22, wherein a multimer (B2) of the compound represented by formula (b) supplied to the reaction system is hydrolyzed to obtain a regenerated compound (B3) represented by formula (b) in the reaction system.

25. The method for producing a cyclic lactam according to claim 24, wherein the regenerated compound (B3) represented by formula (b) is used as the compound represented by formula (b) supplied to the reaction system.

26. An apparatus for producing a cyclic lactam, having the following equipment for carrying out the method for producing a cyclic lactam according to any one of claims 1 to 25:
(1) a reactor for heating a mixture A comprising the polyamide resin comprising a repeating unit represented by formula (a) and the compound represented by formula (b) to carry out a depolymerization reaction and produce a cyclic lactam, thereby obtaining a mixture B comprising the compound represented by formula (b) and the cyclic lactam;
(2) a container for accommodating the cyclic lactam evaporated from the mixture B in the reactor; and
(3) a flow path connecting the reactor and the container, for delivering the evaporated cyclic lactam from the reactor to the container.

27. The apparatus for producing a cyclic lactam according to claim 26, further comprising a pressure regulating device for maintaining the pressure in the reactor, the container, and the flow path at 202.6 kPa or less.

28. The apparatus for producing a cyclic lactam according to claim 27, wherein the pressure regulating device is connected to at least one selected from the group consisting of the reactor, the container, and the flow path.

29. The apparatus for producing a cyclic lactam according to claim 26, having a heating part for maintaining the temperature in the flow path at or above the melting point of the cyclic lactam.

30. The apparatus for producing a cyclic lactam according to claim 26, having an apparatus for removing moisture from the reactor.
